# EUROPEAN PATENT APPLICATION

(11) **EP 0 933 460 A1**
(43) Date of publication of application: **04.08.1999**
(21) Application number: 99101464.8
(22) Date of filing: 27.01.1999
(51) Int. Cl.: D04H 3/04, D04H 3/16, D04H 13/00

(54) **Extendable nonwoven fabric and method for producing the same**

(30) Priority: 30.01.1998 JP 3424298
(71) Applicant: Nippon Petrochemicals Company, Limited, Tokyo 100-8530 (JP); POLYMER PROCESSING RESEARCH INSTITUTE LIMITED, Tokyo 173 (JP)
(72) Inventor: Kurihara, Kazuhiko, Tokyo 175-0082 (JP); Yazawa, Hiroshi, Kunitachi-shi, Tokyo 186-0002 (JP); Yabe, Kazuhiro, Tokyo 152-0003 (JP); Umejima, Shin'ichi, Kawasaki-shi, Kanagawa 214-0021 (JP); Kimura, Masato, Fujisawa-shi, Kanagawa 252-0816 (JP)
(74) Representative: Strehl Schübel-Hopf & Partner

(57) **Abstract**

An extendable nonwoven fabric which comprises a web of long filaments of mainly 3 µm to 15 µm in diameter that is prepared by stretching at least in one direction at a stretching ratio of 3 or more, and which fabric is 150% or more in elongation when it is elongated at right angles to the direction of stretching, a composite extendable nonwoven fabric which is made by laminating an elastomeric material with the extendable nonwoven fabric, and a method for producing the extendable nonwoven fabric, in which the extendable nonwoven fabric is low in basis weight, large in the degree of elongation, and excellent in strength and touch feeling and suitably used as the elastic material for sanitary fittings.

## Description

### BACKGROUND OF THE INVENTION

### (1) Field of the Invention

This invention relates to an extendable nonwoven fabric and a method for producing the same.

More particularly, the invention relates to an extendable nonwoven fabric having an elongation property of 150% or more in the direction at right angles to the stretching direction that is obtained by stretching a web which is prepared by melt-blow spinning or span-bond spinning, and a method for producing the same. The above extendable nonwoven fabric is especially useful for producing sanitary fittings or the like.

### (2) Prior Art

In the conventional art, there are sold widely various kinds of sanitary fittings such as diapers and pads for incontinence of flat type or side-flap type, the so-called trunks-type ones. The trunks-type products have both side flaps to be fitted to waist and openings for receiving thighs. These trunks-type diapers and pads must be well fitted to the thighs and waist when they are put on the body and during the wearing. Accordingly, these goods are made by using materials having elasticity. The nonwoven fabric employed as such an elastic material is required that it can be elongated for about 150% or more.

As the nonwoven fabric used in the field of this kind, there is one material which is made by water jet entangling of a short fiber web. However, when the elongation property is enlarged, the surface strength is lowered and short fibers are liable to fall off. In addition, the unit price of the product of this kind is relatively low, so that the reduction of production cost is regarded as important. However, the above water jet entangling method has a problem in that the production cost is high because its productivity is low.

Some prior art in this field is disclosed in Japanese Laid-Open Patent Publication No. Hei 9-132856. In the method as disclosed in this patent gazette, a spunbond nonwoven fabric is lightly subjected to embossing (thermal point bonding) and it is then stretched longitudinally with allowing the reducing of width, and further the embossing is applied thereto. In this method, the process is complicated because the embossing is done twice, so that the working efficiency is low. In addition, in order to maintain good elongation property, the embossing conditions must be delicately controlled, so that the productivity is low. Furthermore, the stretching is accompanied by the contraction in the transverse direction to reduce the width and the shrinkage of side-edges is large, so that the product is not uniform in the width direction and the yield rate is low.

Technical arts in this field are also disclosed in Japanese Laid-Open Patent Publication Nos. Hei 9-279453 and 9-279460, however, they do not employ the so called close gap stretching in true meaning and they are also accompanied by reduction of width in stretching. In this regard, there remain problems in the uniformity and yield rate in stretching process just like the method disclosed in the foregoing Japanese Laid-Open Patent Publication No. Hei 9-132856.

The object of inventions in the above Japanese Laid-Open Patent Publication Nos. Hei 9-279453 and 9-279460 are the same as those of the present invention in that they relate to the production of elastic nonwoven fabric for sanitary fittings. However, the elasticity is imparted by only the nonwoven fabric itself without combining with an elastomeric material. Therefore, several operation conditions are required.

The extendable nonwoven fabric according to the present invention is different from these materials in view of the fact that the nonwoven fabric of this invention is suitable for combining with an elastomeric material by superposing. It is not necessary that the extended material retracts to the former condition. The ratio of stretching is 1.4 to 4.0 in the above-mentioned patent gazettes, however, in view of the fact that the stretching process causes a reduction in width, the ratio would correspond to 2 or less when expressed by the specific ratio as defined in the present invention.

In order to produce a nonwoven fabric having a larger elongation property, it is necessary that filaments are sufficiently entangled together. For this purpose, a larger number of filaments is required. The filaments of ordinary spunbond nonwoven fabric are fine in diameters, i.e. it is mainly composed of filaments of 20-25 µm in diameter. Therefore, when fabrics of the same basis weight are compared, the number of filaments is smaller in the ordinary nonwoven fabric and an unevenness in thickness is liable to occur and the unevenness in elongation property is also large. Therefore, the feeling to the touch of the ordinary nonwoven fabric is undesirable and the waterproofness is not good. For this reason, the ordinarily used spunbond nonwoven fabric is 60-80 g/m² in basis weight. If the basis weight is large as such, not only the cost is raised but also the elastic portions of products are too stiff and the feeling in wearing is not good.

On the other hand, the ordinary melt blown nonwoven fabrics are composed of filaments of 2-4 µm in diameter, however, each filament is too weak and elongation property is low, so that the stretching is difficult to be done. Even when they are stretched, it is not possible to obtain a highly stretched nonwoven fabric like the extendable nonwoven fabric of the present invention,

The nonwoven fabric according to the present invention is used by superposing (laminating) with an ordinary elastomeric material. Furthermore, the extendable nonwoven fabric of this invention is especially suitable for the elastic portions of sanitary fittings.

### BRIEF SUMMARY OF THE INVENTION

In view of the conventional art as described above, it is the object of the present invention to provide a nonwoven fabric and a method for producing the same, which fabric is low in basis weight and large in elongation property, in addition, excellent in strength and feeling to the touch and is suitable for use as an elastic material for producing sanitary fittings.

The inventors of the present invention have carried out extensive investigations in order to solve the above-described problems in the prior art. As a result, an extendable nonwoven fabric having the following characteristic features, a composite extendable nonwoven fabric, an elastic material prepared by using the same for sanitary fittings, and method for producing them has been provided.

The present invention provides an extendable nonwoven fabric which comprises a web of long filaments of 3-15 µm, inclusive, in diameter prepared by stretching at least in one direction at a stretching ratio of 3 or more, and which is 150% or more in elongation when it is elongated at right angles to the direction of stretching.

In the above nonwoven fabric, when the web is extended, its extension does not return to the original state substantially and the basis weight of the web is uniform in the direction at right angles to stretching. As the material to be stretched, melt blown nonwoven fabric or spunbond nonwoven fabric can be used.

As the material for the foregoing long filaments, any of thermoplastic resins such as polyolefin resin e.g. polyethylene and polypropylene, polyester resin, polyamide resin, polyvinyl alcohol resin, can be used.

The long filaments are prepared by stretching near the softening point which is higher than usual stretching temperature. The length of the long filaments is more than several hundred millimeters. It is possible to mix unstretched filaments of 10 µm or more in diameter into the above extendable nonwoven fabric so that the non-stretched filaments function as an adhesive agent or a gluing agent.

The present invention further relates to a composite extendable nonwoven fabric. It is made by laminating (a) an elastomeric material with (b) an extendable nonwoven fabric of a web of long filaments of 3 to 15 µm in diameter, which web is made by stretching at least in one direction at a stretching ratio of 3 or more and when the web is elongated at right angles to the stretching direction, it can be extended by 150% or more.

The materials used for making this elastomeric material for the composite extendable nonwoven fabric may be made of elastomer of thermoplastic resins such as polyolefin, polyester, polyamide and polyurethane; or elastomer of synthetic rubber or natural rubber. The elastic members for sanitary fittings made by using the composite extendable nonwoven fabric of this kind are also included in the present invention.

Furthermore, the method for producing the extendable nonwoven fabric of the present invention is characterized in that molten filaments which are extruded from the fine pore nozzles of a spinning apparatus, are cooled by cooling medium, the cooled filaments are led onto a conveyor to form a nonwoven fabric, and the nonwoven fabric is then stretched at a ratio of 3 or more at least in one direction.

The stretching in this method can be carried out through constant width close gap stretching method or transversely stretching method. The temperature of stretching of the filaments is close to the softening point which is higher than the usual stretching temperature. For example, in the case of polypropylene filament, the stretching is done at a temperature of 120 to 160°C. The filaments of polyethylene terephthalate can be stretched at a temperature of 90 to 130°C. By subjecting the filaments to stretching or heat treatment at a temperature close to their softening point, partial fusion occurs by receiving heat.

In the following, the present invention will be described in more detail.

### DETAILED DESCRIPTION OF THE INVENTION

The extendable nonwoven fabric of the present invention comprises filaments of mainly 3-15 µm, preferably 4-12 µm in diameter. These filaments are different from filaments of spunbond nonwoven fabric of 25 µm in diameter or thin filaments of less than 4 µm in diameter. Excessively thin filaments is not good in stretching property and low in strength and elongation.

The method for producing the spunbond nonwoven fabric or melt blown nonwoven fabric used for the extendable nonwoven fabric of the present invention, and the stretching method for them are disclosed in Japanese Laid-Open Patent Publication Nos. Hei 8-174764 and Hei 10-204767 and Japanese Patent Publication No. Hei 3-36948, the invention of which were made by the present inventors. However, the method is not restricted to these.

The ordinary nonwoven fabrics made by spunbond method or melt blow method are inferior in stretchability, so that the high ratio stretching as that in the present invention is hardly carried out. However, by employing the spinning method and stretching method as disclosed in the foregoing patent gazettes, the stretching at higher ratio can be made possible. However, the extendable nonwoven fabric of the present invention and method for producing the same are not restricted to these methods.

The extendable nonwoven fabric of the present invention is made by stretching in a longitudinal direction or transverse direction. The term "stretching" herein referred to means that filaments are accumulated onto a conveyor to form a web and the web is stretched in the longitudinal direction or in both the longitudinal and transverse directions under tension. Thus the drafting of filaments prior to the settling on a conveyor is not included.

The term "stretching" means to elongate substantially the constituting filaments, which is different from the term "widening" to extend by the movement of filaments from one another. In this meaning, the term "stretching" used in the foregoing Japanese Laid-Open Patent Publication No. Hei 9-132856 is different because the width is reduced by stretching and the ratio of stretching is low. In the example of this patent gazette, the basis weight after stretching is larger than the basis weight before the stretching.

Also in the inventions as disclosed in Japanese Laid-Open Patent Publication Nos. Hei 9-279453 and 9-279460, the stretching accompanied with the reduction of width, is carried out, and the ratio of stretching corresponds to a value of 2 or less in view of the definition of the present invention.

According to the present invention, the ratio of stretching is substantially 3 or more, and preferably more than 4. It should be noted that the ratio of stretching used in the present invention is the value of the quotient of the basis weight before stretching divided by the basis weight after stretching.

The extendable nonwoven fabric of the present invention is characterized in that the elongation property is large. When the filaments made of synthetic resin is stretched, the elongation property in the direction of stretching is generally reduced. However, in the present invention, it is characterized in that the elongation property in the direction at right angles to the stretching direction, is large. As the elongation property in this meaning, 150% or more, preferably more than 200%, and more preferably more than 300%, is required for the extendable nonwoven fabric before the lamination with the elastomeric material.

Furthermore, not only that the large elongation property is required but also the stress at 150% elongation must be 300 g or less, preferably 200 g or less and more preferably 150 g or less per 1 inch width. If the stress in elongation is high, it is not suitable for practical uses because the tightening force in wearing is too large. Still further, it is not necessary that the extendable nonwoven fabric of the present invention recovers the original shape after its elongation as described in the foregoing. This is because of the fact that the extendable nonwoven fabric of the present invention is generally used by laminating with an elastomeric material.

In this invention, the long filament nonwoven fabric made by melt blow spinning or spunbond spinning, is used. The ordinary melt blown nonwoven fabric is composed of fine filaments of less than 4 µm in diameter, so that filaments are cut in the length of several ten millimeters to several hundred millimeters. In the present invention, however, the diameter of filaments is larger than them, so that torn-off filaments are less and the length of filaments is more than several hundred millimeters. In this meaning, the filaments of this invention are longer as compared with the conventional melt blown filaments. Accordingly, the term "long filaments" in the present invention means that the length of the majority of constituent filaments is several hundred millimeters or more but it does not mean that the length of filaments is limitless.

The melt blow spinning referred to in the present invention is the one generally used for the preparation of melt blown nonwoven fabric. In this melt blow spinning method, the filaments paid out from nozzles are blown by hot air to form many thin filaments and they are accumulated onto a conveyor to obtain a nonwoven fabric.

In the spunbond spinning method, it is difficult to produce the filaments of 20 µm or less in diameter with a high productivity, however, it is relatively easy in the melt blow spinning. Therefore, the melt blow spinning is suitable for the working of the present invention.

The melt blow spinning is suitable for obtaining fine filaments of 4 µm in diameter which are difficultly obtained in the ordinary spinning method. However, such fine filaments are low in strength and are liable to be damaged, so that the ratio of stretching cannot be raised.

In the ordinary melt blow spinning, the large denier filaments of 8 µm or more in diameter before stretching, can be formed by combining such operation conditions that the diameter of nozzles are enlarged, the temperatures of fused polymer and hot air are lowered and the quantity of hot air is reduced so as to increase the quantity of extrusion. However, when melt blow spinning of high denier filaments (8-20 µm in diameter) is done under ordinary conditions, the filaments are not cooled enough when they are thrown onto a conveyor because hot air is used and, in addition, spinning speed is high in this method, so that the filaments adhere by fusion. In addition, the filaments are influenced by heat, so that the intended nonwoven fabric of this invention having good stretching property cannot be obtained. In the melt blow spinning of the present invention, the filaments are cooled by blowing cooled air or air containing aqueous mist. With this cooling operation, it is possible to produce a soft and stretchable nonwoven fabric from the spun filaments.

In the spunbond spinning in the present invention, the filaments extruded from nozzles are introduced into a suction box by cooling air and they are accumulated on a conveyor. In the ordinary spunbond spinning method, spun filaments are subjected to drafting with giving possibly effective cooling in order to raise the strength of filaments. However, in the present invention, the strength of filaments is not important on this stage because the strength of filaments can be improved by subsequent stretching process, meanwhile the spinning of finer filament is rather preferable. In the ordinary spunbond nonwoven fabric, the diameter of filaments is 20-30 µm, however, the intact filaments are less entangled. Hence, it is necessary to use a nonwoven fabric of larger basis weight in order to obtain the nonwoven fabric which can be largely stretched in one direction.

Therefore, in the practice of the present invention, it is preferable to raise the ratio of drafting and to form finer filaments by positively subjecting the portion near the nozzles for spunbond nonwoven fabric to heating or heat-retaining at a temperature above the melting point of fused polymer.

In the present invention, the nonwoven fabric is stretched at least in one direction. The stretching is divided into longitudinal stretching and transverse stretching.

Several kinds of longitudinally stretching methods for films and nonwoven fabrics can be employed as the above longitudinally stretching method, in which those disclosed in the foregoing Japanese Laid-Open Patent Publication Nos. Hei 8-174764 and 10-204767 and Japanese Patent Publication No. Hei 3-36948, are suitable. Among them, the most suitable method for the method of the present invention is a roll close gap stretching method.

The roll close gap stretching method is carried out such that the rolls before and after the stretching are brought into closely spaced-apart relation to each other so that the stretching is done in the narrow gap between the two rolls. The space between rolls is different according to the width of a web, it may be at most a half, preferably less than one fifth and more preferably less than one tenth, relative to the width of the web to be stretched.

In the close gap stretching like this, the width of the web is not narrowed and the basis weight and stretching effect in the direction of the width of web can be maintained uniformly. Furthermore, the web is stretched with accompanying substantial molecular orientation without the dropping off of filaments. In addition, the lowering in strength in the direction at right angles to the stretching direction is also small, therefore it is quite suitable for the purpose of the present invention.

In the present invention, the ratio of stretching is 3 or more and preferably more than 4. If the ratio of stretching is less than 3, the elongation in the direction at right angles to the stretching direction is small and the stress of stretching is large. However, too high ratio of stretching is not always desirable, so that the suitable ratio of stretching of 3 or more must be selected.

The ratio of stretching is represented by dividing a basis weight (g/m²) of a unit area (usually 250 mm X 250 mm) of a web before stretching by the similar basis weight of the web after the stretching. This value substantially means the ratio of close gap stretching.

This ratio of stretching does not always represent the ratio of stretching of each filament like that of long filament yarn.

As the above method for transversely stretching, various transversely stretching methods for films and nonwoven fabrics are employed. As the methods for working in the present invention, the methods disclosed in Japanese Patent Publication No. Hei 3-36948, and Japanese Laid-Open Patent Publication Nos. Hei 2-269859 and 2-242960, are suitable. Among them, the pulley transversely stretching method is especially suitable for the present invention because it is convenient and economical. By the way, the transversely stretched nonwoven fabric is sometimes more suitable when it is used as a material for sanitary fittings.

In the above-described stretching process, it was understood that a nonwoven fabric in which the elongation is larger in one direction can be obtained by subjecting it to the stretching or heat treatment at a temperature above the conventional stretching temperature. This is due to the fact that, in the method of stretching of the present invention, all the filaments of the nonwoven fabric are not stretched but the filaments are stretched partially, that is, the filaments in the direction at right angles to the stretching direction are not elongated and they remain as they stand in the stretched nonwoven fabric. The filaments which are not stretched substantially, are partially fused by the heat of stretching or heat treatment and such parts form micro bonding points when the nonwoven fabric is extended in the direction at right angles to the stretching direction. Accordingly, in order to produce this bonding effect, the stretching or heat treatment must be done at a temperature near the softening point and higher than the ordinary stretching temperature. Therefore, in the case of quenched (rapidly cooled) polypropylene nonwoven fabric, it is generally stretched at about 90-110°C, however, the nonwoven fabric of the present invention is preferably stretched or heat-treated at 120-160°C. Likewise, in the case of rapidly cooled polyethylene terephthalate nonwoven fabric, it is generally stretched at about 80-90°C, however, the nonwoven fabric of the present invention is preferably stretched or heat-treated at 90-130°C.

In the conventional non woven fabric, products of this kind are generally made with finally applying thermal point bonding. However, the treatment with thermal point bonding is not necessary in the present invention. Therefore, not only the production process can be simplified but also the advantages in softness and even thickness of the fabric are not lost. However, in some usages, products can be applied with the thermal point bonding lightly.

The nonwoven fabric stretched according to the present invention is a flat sheet-like material as it stands, so that the softness is not satisfactory sometimes. In order to give softness to the product of nonwoven fabric, it is sometimes effective to treat it with Micrex equipment or to apply Sanforized finish or other softening treatment.

When lamination or bonding of the nonwoven fabric of the present invention with an elastomeric material or other kind of nonwoven fabric is done, a treatment with an anchor coating agent or an adhesive agent is often applied.

The polymers used for making the nonwoven fabric of the present invention, are exemplified by thermoplastic resins of polyolefin resins such as polyethylene and polypropylene, polyester resin, polyamide resin, and polyvinyl alcohol resin. Among them, the polypropylene is most suitable as the polymer used for the nonwoven fabric of the present invention because it is excellent in spinning property, water repelling property and heat-sealing property.

The above polymers can be used singly, however, it is also possible to use a mixed yarn composed of several kinds of filaments or a conjugate yarn made of different kinds of polymers as disclosed in International Patent Publication WO 96/17121 invented by the present inventors.

In the present invention, the extendable nonwoven fabric is used by laminating with elastomeric material. The elastomeric material includes elastomers made of thermoplastic resins such as polyolefins, polyesters, polyamides and polyurethanes and natural and synthetic rubbers. Among them, the polyurethane elastomer is most suitable because it can be stretched at a high ratio, in addition, the stress in the stretching is small.

The forms of the elastomeric materials include films, nonwoven fabrics, and foamed materials. As the method for laminating the elastomer with the extendable nonwoven fabric, extrusion lamination, bonding with an adhesive agent and coating with elastomer solution or elastomer emulsion can be employed.

In the following, the present invention is described in more detail.

### EXAMPLES

### 〈Methods for spinning and stretching〉

The methods as disclosed in Japanese Laid-Open Patent Publication Nos. Hei 8-174764 and 10-204767 and Japanese Patent Publication No. Hei 3-36948 were employed.

### 〈Polymers Used〉

| | |
|---|---|
| Polypropylene (PP): | MFR = 200 g/10 min |
| Polyethylene terephthalate (PET): | IV = 0.67 |

| (Spinning Method) | |
|---|---|
| Melt blowing method: | Nozzle dia. = 0.5 mm |
| | Temperature of die and hot air = 300°C |
| Spunbond method: | Nozzle dia. = 0.3 mm |
| | Temperature of die = 280 °C, the lower parts of nozzles were heated with infrared heater |

### 〈Measuring Conditions for Physical Properties〉

- Width of nonwoven fabric sample:: 25 mm
- Distance of chucks:: 50 mm
- Tensile speed:: 100 mm/min

Other conditions were set according to JIS L 1096 "Test method for non-woven fabric made of filament yarn"

### 〈Examples 1 and 2〉

Melt blow spinning was done using PP, which was followed by longitudinal stretching under ordinary stretching conditions in Example 1 and under a higher temperature condition in Example 2. The results are shown in Table 1.

### 〈Example 3〉

Spray nozzle spinning was done using PP, which was followed by transverse stretching of the obtained nonwoven fabric. The results are also shown in Table 1.

### 〈Example 4〉

Spunbond spinning was done using PP, in which the portion near the spinning nozzles was heated to 300 °C to obtain nonwoven fabric of fine denier. It was then longitudinally stretched. The results are shown in Table 1.

### 〈Examples 5 and 6〉

Melt blow spinning was done using PP, which was followed by longitudinal stretching under ordinary conditions in Example 5 and at a higher temperature in Example 6. The results are shown in Table 1.

### 〈Example 7〉

Melt blow spinning was done using PP to obtain the extendable nonwoven fabric like that in Example 1. This was laminated with urethane resin by extrusion lamination. The results are shown in Table 2.

### 〈Example 8〉

Melt blow spinning was done using PET to obtain a stretched nonwoven fabric like that in Example 5. This was laminated with urethane resin by extrusion lamination. The results are shown in Table 2.

### 〈Example 9〉

Melt blow spinning was done using PET to obtain a stretched nonwoven fabric like that in Example 5. This was bonded with urethane nonwoven fabric (trademark: Espanione made by Kanebo Ltd.) using urethane emulsion adhesive agent (trademark: Super Flex E 2000 made by Daiichi Kogyo Seiyaku Co., Ltd.). The results are shown in Table 2.

**Table 1**

| Example | 1 | 2 | 3 | 4 | 5 | 6 |
|---|---|---|---|---|---|---|
| Polymer for Spinning | PP | PP | pp | pp | PET | PET |
| Method of Spinning | Melt blow Mist cooling | Melt blow Mist cooling | Spray nozzle spinning | Spunbond Spray cooling | Melt blow Mist cooling | Melt blow Mist cooling |
| Method of Stretching | L R CGS 105°C | L R CGS 133°C | T P S HA 110°C | L R CGS 110°C | L R CGS 86°C | L R CGS 97°C |
| Ratio of Stretching | 5.4 | 6.2 | 5.1 | 3.8 | 4.5 | 5.3 |
| Basis Weight Before Stretching /After Stretching | 103.1 /19.1 | 95.5 /15.4 | 106.1 /20.8 | 107.2 /28.2 | 92.3 /20.5 | 88.0 /16.6 |
| Thickness of Main filament (µm) After Stretching | 5.5 | 4.8 | 6.1 | 9.5 | 5.8 | 4.8 |

| Properties in Direction at Right Angles to Stretching | | | | | | |
|---|---|---|---|---|---|---|
| Stress at 150% Elongation (g/in) | 143 | 101 | 184 | 285 | 183 | 97 |
| Stress at Break Down (g/in) | 495 | 362 | 387 | 544 | 580 | 393 |
| Elongation at Break Down (%) | 281 | 375 | 194 | 187 | 302 | 381 |

### Notes for Table 1

- L R CGS:: Longitudinal roller close gap stretching
- T P S:: Transverse pulley stretching
- HA:: Hot air

**Table 2**

| Example | 7 | 8 | 9 |
|---|---|---|---|
| Stretched Nonwoven Fabric | Example 1 | Example 5 | Example 5 |
| Kind of Elastomer | Urethane film | Urethane Film | Urethane Nonwoven fabric |
| Bonding Method | Extrusion lamination | Extrusion lamination | Adhesive agent |

| Properties in Direction at Right Angles to Stretching | | | |
|---|---|---|---|
| Stress at 150% Elongation (g/in) | 295 | 322 | 294 |
| Stress at Break Down (g/in) | 570 | 599 | 575 |
| Elongation at Break Down (%) | 272 | 231 | 220 |

### ADVANTAGES IN THE INVENTION

The present invention has the following advantages in view of the prior art.
(1) The extendable nonwoven fabric of the present invention is excellent in stretching property as compared with the ordinary melt blown nonwoven fabric, soft and good in feeling to the touch due to the small diameter of its filaments as compared with the spunbond nonwoven fabric and has water repelling property. Furthermore, the extendable nonwoven fabric of the invention is uniform in thickness because the number of filaments in a unit area is large and the distribution of filaments is even.
(2) The material made by stretching an ordinary spunbond nonwoven fabric shows little entanglement of filaments because the number of filaments is small and, in view of the micro bonding structure, it is different from the extendable nonwoven fabric of the present invention which has a large number of micro bonding points. Accordingly, the ordinary nonwoven fabric must have a basis weight of 50 g/m², preferably 80 g/m².
   On the other hand, the number of filaments of the nonwoven fabric of the present invention is large because the diameter of filaments is smaller and unstretched filaments form partial bonding points. Therefore, the basis weight is 30 g/m² or less, preferably less than 20 g/m², and more preferably 15 g/m², so that the extendability is high. In addition, the production cost is low and the material is soft and good in feeling to the touch.
   Furthermore, the basis weight of nonwoven fabric of the present invention before stretching is large, so that the stretching can be done without causing an unevenness. In addition, the stretching is done at a high ratio, so that the obtained product is uniform in thickness and basis weight in Spite of its low basis weight.
(3) In the present invention, because the filaments are stretched, the strength is large. The stretched filaments are flexible to give excellent touch feeling. This effect is improved because the stretching is done at a higher ratio to make the filaments thinner.
(4) As the nonwoven fabric of the present invention is composed of long filaments, they are hardly fallen off and the effect of stretching can be enhanced.
(5) In the present invention, thermal point bonding of two steps is not necessary, so that the production process can be simplified. In addition, shrinkage in the direction at right angles to the stretching direction is not caused, so that the productivity is raised. Furthermore, in the product free from the thermal point bonding, the degree of elongation is large and the touch feeling is good since the nonwoven fabric is made of the fine filament network.

## Claims

1. An extendable nonwoven fabric comprising a web of long filaments having a diameter of mainly not less than 3 µm to not more than 15 µm that is prepared by stretching at least in one direction at a stretching ratio of 3 or more, said fabric having an elongation of 150% or more when it is elongated at right angles to the direction of stretching.

2. An extendable nonwoven fabric as claimed in claim 1, wherein, when said web is extended, its extension does not substantially return to the original state.

3. An extendable nonwoven fabric as claimed in claim 1 or 2, wherein the basis weight of said web is uniform in the direction at right angles to the stretching direction.

4. An extendable nonwoven fabric as claimed in any of the claims 1 to 3, wherein said nonwoven fabric before stretching is a melt blown nonwoven fabric or spunbond nonwoven fabric.

5. An extendable nonwoven fabric as claimed in any of the claims 1 to 4, wherein said long filaments are made of a thermoplastic resin selected from the group consisting of polyolefin resin such as polyethylene and polypropylene, polyester resin, polyamide resin, and polyvinyl alcohol resin.

6. An extendable nonwoven fabric as claimed in any of the claims 1 to 5, wherein said long filaments are prepared by stretching near the softening point at a temperature higher than the usual stretching temperature.

7. An extendable nonwoven fabric as claimed in any of the claims 1 to 6, wherein the length of the most of said long filaments is more than several hundred millimeters.

8. An extendable nonwoven fabric as claimed in any of the claims 1 to 7, wherein non-stretched filaments having a diameter of 10 µm or more are mixed into the above extendable nonwoven fabric wherein the non-stretched filaments function as an adhesive agent or a bonding agent.

9. A composite extendable nonwoven fabric which is made by laminating
(a) an elastomeric material with
(b) an extendable nonwoven fabric as claimed in any of the claims 1 to 8.

10. A composite extendable nonwoven fabric as claimed in claim 9, wherein said elastomeric material is made of one member selected from the group consisting of an elastomer of thermoplastic resins of polyolefin, polyester, polyamide and polyurethane; and an elastomer of synthetic rubber and natural rubber.

11. An elastic member for sanitary fittings which is made by using said composite extendable nonwoven fabric as claimed in claim 9.

12. A method for producing an extendable nonwoven fabric which comprises the steps of:
cooling the molten filaments extruded from the fine orifices of nozzles of a spinning apparatus with a cooling medium,
introducing the cooled filaments onto a conveyor to form a nonwoven fabric, and
stretching said nonwoven fabric at a ratio of 3 or more at least in one direction.

13. A method for producing an extendable nonwoven fabric as claimed in claim 12, wherein said stretching is constant width close gap stretching or transverse stretching.

14. A method for producing an extendable nonwoven fabric as claimed in claim 12 or 13, wherein the temperature of stretching of said filaments is close to the softening point which is higher than the usual stretching temperature.

15. A method for producing an extendable nonwoven fabric as claimed in any of the claims 12 to 14, wherein said filaments are made of polypropylene and the stretching is carried out at a temperature of 120 to 160°C.

16. A method for producing an extendable nonwoven fabric as claimed in any of the claims 12 to 14, wherein said filaments are made of polyethylene terephthalate and the stretching is carried out at a temperature of 90 to 130°C.

17. A method for producing an extendable nonwoven fabric as claimed in any of the claims 12 to 16, wherein said filaments are subjected to stretching or heat treatment at a temperature close to their softening point.
